# EUROPEAN PATENT APPLICATION

(11) **EP 3 929 580 A1**
(43) Date of publication of application: **29.12.2021**
(21) Application number: 20181633.7
(22) Date of filing: 23.06.2020
(51) Int. Cl.: G01N 33/24, G06F 17/40

(54) **METHOD FOR DETERMINING SOIL TEXTURE**

(71) Applicant: Yara International ASA, 0277 Oslo (NO)
(72) Inventor: PRICE, Nathan, 555109 Singapore (SG); SAUTON, Vincent, 149157 Singapore (SG)
(74) Representative: AWA Sweden AB

(57) **Abstract**

The present invention relates to a method for determining soil texture, comprising: placing soil (32) and a water-based liquid (34) in a substantially transparent container (16); mixing the soil and water-based liquid in the substantially transparent container; allowing particles of the soil to separate into layers (36a-c) by gravity; capturing an image (38) of the layers in the substantially transparent container by means of a camera (12) of a mobile computing device (10); and an app (30) of the mobile computing device detecting the layers in the captured image and determining the texture of the soil based on the detected layers.

## Description

### Technical field

The present invention relates to a method for determining soil texture. The present invention also relates to a mobile computing device, a computer program product, a computer-readable storage medium, and an electrical signal.

### Background

Soil texture has a strong impact on fertilizer and irrigation management.

Determining the soil texture can for example be done by lab analysis. In countries where soil chemical/lab analysis is a routine test, the soil texture is systematically determined. For this reason, extensive texture databases already exist in those countries. However, for smallholder farmers, such information may be very poor or non-existent.

Another way of determining soil texture includes shaking water and soil in a jar and letting the sand, silt, and clay separate by gravity. This technique requires the user to measure the total height of the soil and the height of each layer (sand, silt, and clay) with the naked eye to determine the percentage of each component. The user then compares the percentages of silt/sand/clay to a so-called soil texture triangle to determine the soil texture.

However, properly measuring the heights, performing the percentage calculations, and/or reading the soil texture triangle could be difficult for users, making this technique (potentially) unreliable.

### Summary of the invention

It is an object of the present invention to overcome or at least alleviate the aforementioned problems.

According to a first aspect of the present invention, this and other objects is achieved by a method for determining soil texture, comprising: placing soil and a water-based liquid in a substantially transparent container; mixing the soil and water-based liquid in the substantially transparent container; allowing particles of the soil to separate into layers (segments) by gravity; capturing an image of the layers in the substantially transparent container by means of a camera of a mobile computing device; and an app of the mobile computing device detecting the layers in the captured image and determining the texture of the soil based on the detected layers.

The present invention is based on the understanding that by capturing an image of the layers using a mobile computing device camera and providing an app on the mobile computing device that detects the layers (using image analysis/recognition) and determines the soil texture, site specific soil texture determination may be simplified, and also the accuracy of the soil texture determination may be improved. That is, a farmer may just capture the image using their smartphone (mobile computing device) and then the app on the smartphone automatically determines the soil texture. The farmer does not need to know how to calculate the aforementioned percentages and how to read the soil texture triangle, and potential errors in manually measuring the height of the layers, calculating the percentages and/or reading the soil texture triangle may be avoided. Furthermore, no expensive equipment is needed, as the farmer may already have a smartphone (i.e. the mobile computing device) on which the app may be downloaded.

The method for soil texture detection could use one or more of per se known edge and corner detection algorithms in image processing (e.g. Sobel operators, Canny edge, Shi-Tomasi).

Detecting the layers in the captured image may include determining the thickness of each layer based on the captured image and determining the percentage of each layer based on the determined thicknesses. The layers typically include a (bottom) sand layer, a(n intermediate) silt layer, and a (top) clay layer. In an example, the determined thickness of the sand layer is 410 pixels, the determined thickness of the silt is 390 pixels, and the determined thickness of the clay layer is 200 pixels, whereby the percentage of sand is 410/(410+390+200)=41%, the percentage of silt is 390/(410+390+200)=39%, and the percentage of clay is 200/(410+390+200)=20%, which corresponds to the soil texture 'loam' (e.g. according to the soil texture triangle).

Detecting the layers in the captured image may include distinguishing at least one delimitation (interface) between the layers by identifying differences in colour between the layers. This may be achieved by looking at the different RGB values of pixels in the image. Based on that, an algorithm may be trained to distinguish (detect) the delimitation or boundary between two layers automatically.

Detecting the layers in the captured image may include distinguishing at least one delimitation (interface) between the layers by identifying differences in texture, in particular granularity, between the layers. This may include image processing to highlight the grain/roughness/granularity, and using an algorithm that picks up the boundaries of each layer by being able to identify each layer. Typically, the sand layer has coarser texture/granularity, the silt layer has "intermediate" texture/granularity, and the clay layer has finer texture/granularity.

Detecting the layers in the captured image based on colour and/or texture makes the layers easy to detect, in that colour and texture are the main characteristics that define the layers.

The app may display on a display of the mobile computing device a shape corresponding to at least a portion of the shape of the substantially transparent container, wherein a user positions and/or aims the mobile computing device such that the substantially transparent container as seen in live view by the camera on the display fits the displayed shape, and wherein the image of the layers in the substantially transparent container is captured as the mobile computing device is so positioned and/or aimed. In this way, the image of the layers may be captured at a predetermined angle relative to the substantially transparent container, which in turn may facilitate and/or increase the accuracy of detecting the layers in the captured image.

The substantially transparent container may comprise a funnel portion emptying into a cylinder portion with a bottom. This may facilitate placing the soil and water-based liquid in the substantially transparent container via the funnel portion, while the percentages of the layers readily can be determined in the cylinder portion. To this end, the volume of soil may be equal to or less than the volume of the cylinder portion of the substantially transparent container. The funnel portion may also provide extra place to mix the soil and water-based liquid, which may facilitate the mixing of for example heavy clay soil.

The method may further comprise the app providing a fertilizer recommendation based at least partly on the determined texture of the soil. The fertilizer recommendation may for example be shown on the aforementioned display of the mobile computing device.

The mobile computing device may be a smartphone or a tablet. The smartphone or tablet may advantageously be a "generic" smartphone or tablet, wherein the above-described functionalities are provided by means of the app stored on the smartphone or tablet.

The method may further comprise using the substantially transparent container being emptied as a rain gauge; capturing an image of rain in the substantially transparent container by means of the camera of the mobile computing device; and the app determining the amount of rain in the substantially transparent container based on the captured image of rain in the substantially transparent container. An advantage of this is that the substantially transparent container can have several purposes, which is good for the environment and avoids single use plastic. Furthermore, using an app on the mobile computing device to determine the amount of rain offers advantages compared to manual rain gauging. For example, push notifications could conveniently be sent to the mobile computing device via the app prompting the user/farmer to check the substantially transparent container based on a weather forecast of rain. In another example, the determined amount of rain could readily be fed back from the app and mobile computing device to a weather forecast provider.

The amount of rain in the substantially transparent container could be determined in the same way(s) as the layers discussed above, and the image of rain in the substantially transparent container may be captured at a predetermined angle relative to the substantially transparent container as also discussed above.

The substantially transparent container may be closed or closable by a cap having at least one of a colour chart known to the app and a size known to the app, wherein the method may further comprise: capturing an image of an object with at least a portion of the cap as background by means of the camera of the mobile computing device; and the app assessing the colour of the object based on the captured image of the object taking into account the known colour chart of the cap and/or assessing the size of the object based on the captured image of the object taking into account the known size of the cap. In the former case, the colour chart (for example a red marker, a green marker, and a blue marker on the cap) should be visible in the captured image. In the latter case, the object is preferably placed on the cap when the image is captured, and the known size (for example a diameter of the cap) should be visible in the captured image. The cap is generally useful for closing the substantially transparent container when mixing the soil and water-based liquid in the substantially transparent container (which typically includes the user/farmer shaking the substantially transparent container with its content), but the present inventors have realized that by programming the colour chart and/or size of the cap into the app, the cap can conveniently also be used for colour and/or size assessment. This (again) expands the use of the hardware, which is good for the environment. Furthermore, by using the app on the mobile computing device to assess the colour of an object, notably soil, the colour assessment can advantageously be combined with the determined soil texture and GPS coordinates from the mobile computing device, which data for example may be used to build a soil colour-texture map, i.e. a dataset which links soil texture - location - colour. Here, if a farmer's location as well as his soil colour (e.g. from a picture of his soil) is known, then the soil texture at the specific farmer site can be assumed.

The colour chart known to the app may be a red marker, a green marker, and a blue marker on the cap. Alternatively it can be a gradient of colour or shades of different colours, for example. For soil, the colour chart could include different shades of black-brown-red-orange-yellow-white, though for practicality there could be one brown shade representing a number of brown shades, for example ten brown shades (of the Munsell soil colour chart), another brown shade representing another ten brown shades, and so on.

Assessing the colour of the object based on the captured image of the object taking into account the known colour chart of the cap may include detecting a colour difference between the colour chart of the cap as depicted in the captured image of the object and the known colour chart of the cap, and using the detected difference as calibration to assess the (true) colour of the object.

According to a second aspect of the present invention, there is provided a mobile computing device for determining soil texture, which mobile computing device comprises a camera, wherein the mobile computing device is configured to: instruct a user of the mobile computing device to capture an image of soil layers in a substantially transparent container using the camera; detect the soil layers in a captured image of the soil layers in the substantially transparent container; and determine a soil texture based on the detected soil layers. This aspect may exhibit the same or similar features and technical effects as the first aspect, and vice versa.

According to a third aspect of the present invention, there is provided a computer program product comprising computer program code to perform, when executed on a computer, the steps of: receiving an image of soil layers in a substantially transparent container captured by a camera of a mobile computing device; detecting the soil layers in the received image; and determining a soil texture based on the detected soil layers. The computer program product may be a non-transitory computer program product. The computer may for example be the aforementioned mobile computing device. The computer program product be the aforementioned app. This aspect may exhibit the same or similar features and technical effects as the first and/or second aspect, and vice versa.

According to a fourth aspect of the present invention, there is provided a computer-readable storage medium comprising the computer program product according the third aspect.

According to a fifth aspect of the present invention, there is provided an electrical signal embodied on a carrier wave and propagated on an electrical medium, the electrical signal comprising the computer program product according the third aspect.

According to a sixth aspect of the present invention, there is provided a mobile computing device comprising the computer-readable storage medium according the fourth aspect.

According to a seventh aspect of the present invention, there is provided a mobile computing device according to the second aspect or the sixth aspect in combination with the substantially transparent container. Hence, the seventh aspect may be expressed as a system comprising the mobile computing device and the substantially transparent container.

### Brief description of the drawings

These and other aspects of the present invention will now be described in more detail, with reference to the appended drawings showing currently preferred embodiments of the invention.
Fig. 1 schematically illustrates a mobile computing device and a container according to aspects of the present invention.
Fig. 2 is a flowchart of a method according to one or more embodiments of the present invention.
Fig. 3 shows an image of soil layers in the substantially transparent container captured by a camera of the mobile computing device of fig. 1.
Fig. 4 illustrates a soil texture triangle.
Fig. 5 illustrates use of the container of fig. 1 as a rain gauge.
Fig. 6 is a top view of a cap for the container.

### Detailed description

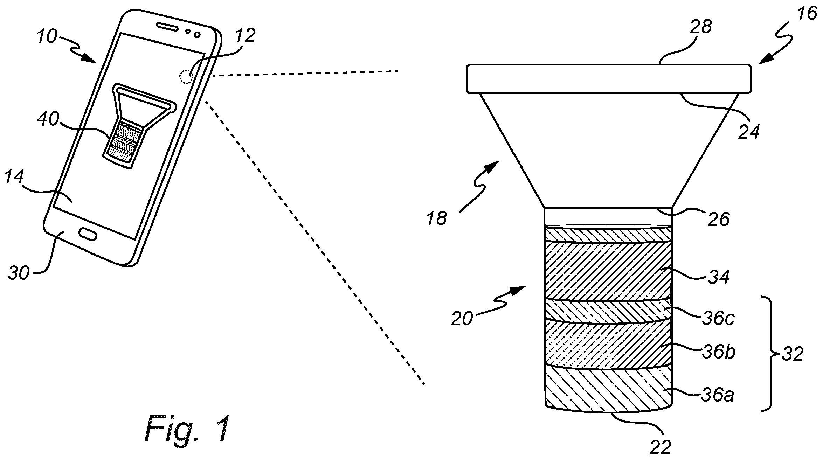

Fig. 1 shows in perspective a mobile computing device 10 for determining soil texture according to an embodiment of the present invention.

The mobile computing device 10 may be handheld and/or portable. The mobile computing device 10 may for example be a smartphone (e.g. an iPhone or an Android phone) or a tablet (e.g. an iPad).

The mobile computing device 10 comprises a (digital) camera 12. The mobile computing device 10 may also comprise one or more of a(n electronic) display 14, a processor, a memory, a storage, a GPS receiver, and wireless communication means.

Fig. 1 also shows in side view a container 16 according to an embodiment of the present invention. The container 16 is a (substantially) transparent container 16. The (substantially) transparent container 16 is preferably close to glass in terms of transparency. The transparent container 16 may for example be made of clear plastics, such as acrylic. No measuring scale has to be provided on the container 16.

The transparent container 16 comprises a funnel portion 18 and a cylinder portion 20 with a bottom 22. The funnel portion 18 may generally have the shape of a(n upside down) truncated cone. The funnel portion 18 has a free end opening 24 which is wider (e.g. greater diameter) than an aperture 26 towards the cylinder portion 20. The cylinder portion 20 is preferably a right circular cylinder portion. The cylinder portion 20 may have a volume in the range of 400-600 cm³. In an example, the cylinder portion 20 has a radius of 3 cm and a height of 18 cm, whereby the volume is approximately 509 cm³. The transparent container 16 is closed or closeable at the free end opening 24 by a cap (lid) 28.

Moving on, the mobile computing device 10 may be configured to perform various specific steps or actions (e.g. S5-S7) detailed in the following by means of an app 30 (computer program product). The app 30 may be downloaded to the mobile computing device 10 via the aforementioned wireless communication means and stored on the aforementioned storage. The app 30 may run or be executed on the mobile computing device 10 using the aforementioned processor and memory.

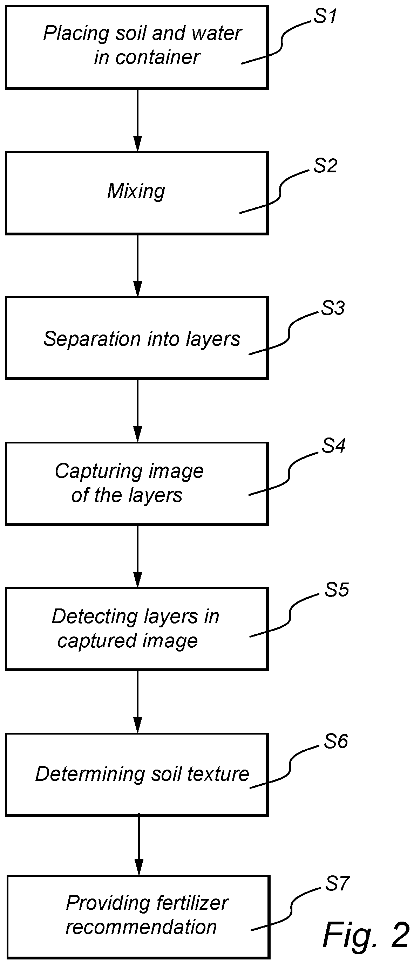

Turning to fig. 2, fig. 2 is a flowchart of a method according to one or more embodiments of the present invention.

At S1, the method includes placing soil 32 and a water-based liquid 34 in the transparent container 16. Specifically, a user (farmer or agronomist) may place an amount of soil 32 in the transparent container 16 and then add the water-based liquid 34 via the free end opening 24. The volume of soil 32 is preferably (somewhat) less than the volume of the cylinder portion 20 of the transparent container 16. Furthermore, the water-based liquid 34 could be water or water with added salt (NaCI), for example.

If the soil 32 is (too) compact, the mobile computing device 10/app 30 may instruct the user to initially dry and then grind the soil sample 32 before placing it in the transparent container 16.

At S2, the method includes mixing the soil 32 and the water-based liquid 34 in the transparent container 16. This may specifically include closing the transparent container 16 with the cap 28, and then the user/farmer shakes the transparent container 16 with its content 32, 34 until all the soil particles are suspended in the water-based liquid 34.

Then the transparent container 16 may rest for some time on a flat surface, for example at least two hours or about 1 day, allowing particles of the soil 32 to separate into layers (segments) 36a-c by gravity, at S3. The layers typically include a bottom sand layer 36a, an intermediate silt layer 36b, and a top clay layer 36c. The (residual) water-based liquid 34 sits above the clay layer 36c.

At S4, the method includes capturing an image 38 of the layers 36a-c in the transparent container 16 by means of the camera 12 of the mobile computing device 10. Before this, the mobile computing device 10/app 30 may instruct the user to capture such an image 38, for example by displaying an appropriate message on the display 14. Furthermore, the app 30 may here display on the display 14 a shape 40 corresponding to at least a portion of the shape of the transparent container 16, wherein the user positions and/or aims the mobile computing device 10 such that the transparent container 16 as seen in live view by the camera 12 on the display 14 fits - as good as possible - the displayed shape 40 (like in fig. 1), and wherein the image 38 is captured as the mobile computing device 10 is so positioned and/or aimed. The shape 40 may for example be an outline of the transparent container 16 as seen from the side, i.e. a line that marks the outer limits of the transparent container 16, or an inverted silhouette of the transparent container 16. The shape 40 may be applied as a(n overlaid) filter in the live view on the display 14.

The app 30 then detects the layers 36a-c in the captured image 38 at S5, and determines the texture of the soil 32 based on the detected layers 36a-c at S6. The determined texture of the soil 32 (soil texture classification), for example 'loamy sand', 'loam', 'silt', 'sandy clay loam' etc., may be displayed on the display 14.

By means of the present invention, site specific soil texture determination may be simplified, and also the accuracy of the soil texture determination may be improved over the fully manual technique described in the background section of the present application. That is, the user/farmer can just capture the image 38 using their smartphone 10 and then the app 30 automatically determines the soil texture. The farmer does not need to know how to calculate percentages of the layer 36a-c and how to read a soil texture triangle, and potential errors in manually measuring the height of the layers 36a-c, calculating the percentages and/or reading the soil texture triangle may be avoided. Furthermore, no expensive equipment is needed, as the farmer may already have the smartphone 10 on which the app 30 may be downloaded.

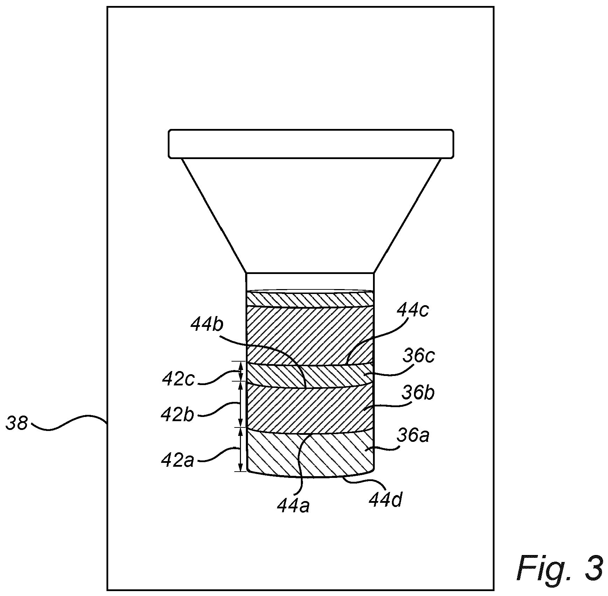

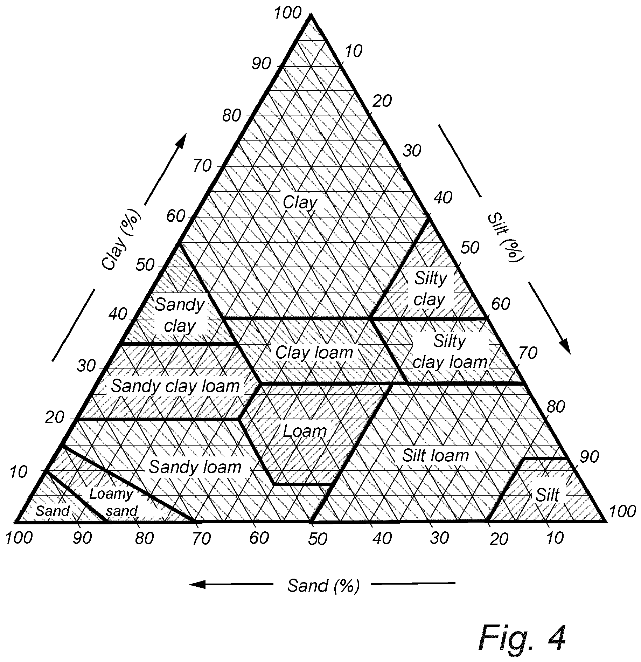

With further reference to fig. 3, detecting the layers 36a-c in the captured image 38 may include determining the thickness 42a-c of each layer 36a-c based on the captured image 38, and determining the percentage of each layer 36a-c relative the complete amount of soil 32 based on the determined thicknesses 42a-c. In an example, the determined thickness 42a of the sand layer is 410 pixels, the determined thickness 42b of the silt is 390 pixels, and the determined thickness 42c of the clay layer is 200 pixels, whereby the percentage of sand is 410/(410+390+200)=41%, the percentage of silt is 390/(410+390+200)=39%, and the percentage of clay is 200/(410+390+200)=20%. Once the percentages have been determined, the app 30 may for example determine the texture of the soil 32 by consulting a soil texture triangle, which soil texture triangle may be programmed into the app 30. In the present example, the percentages sand 41%, silt 39%, and clay 20% correspond to the soil texture 'loam'. An example of a soil texture triangle is shown in fig. 4.

Specifically, detecting the layers 36a-c in the captured image 38 may include distinguishing the delimitations 44a-b between the layers 36a-c by identifying differences in colour and/or texture (granularity) between the layers 36a-c. Furthermore, the upper boundary 44c of the top layer 36c may be detected by identifying difference in colour and/or texture between the layers 36c and the (residual) water-based liquid 34. Furthermore, the lower boundary 44d of the bottom layer 36a may be detected by detecting the bottom 22 of the transparent container 16 directly and account for the material thickness of that bottom 22.

At S7, the method may further comprise the app 30 providing a fertilizer recommendation based at least partly on the determined texture of the soil 32. For example on a sandy soil, the fertilizer recommendation may be to apply a small quantity of fertiliser more regularly in order to avoid it to percolate. In a clay soil, the fertilizer recommendation may be a less frequent but larger fertilization. The fertilizer recommendation may for example be shown on the display 14 of the mobile computing device 10.

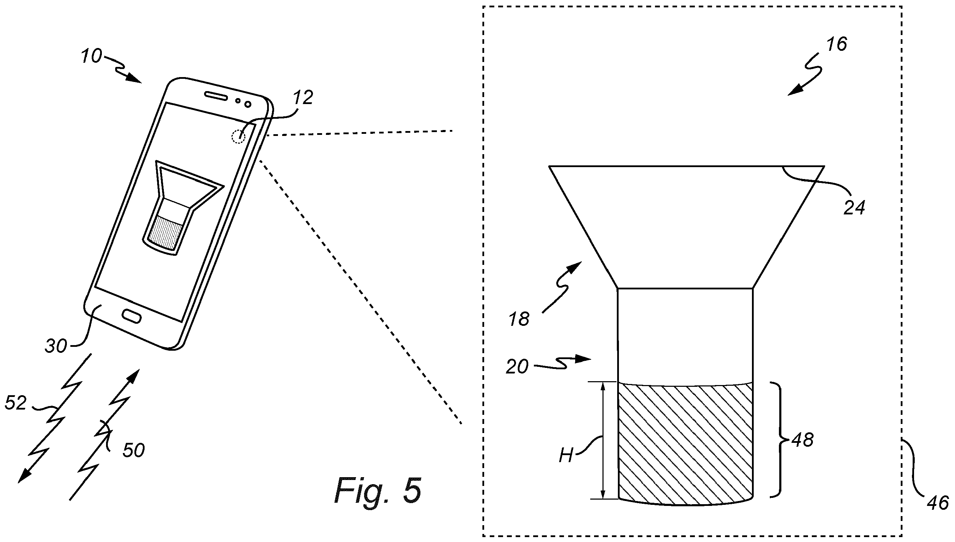

Turning to fig. 5, the transparent container 16 being emptied and without the cap 28 may further be used as a rain gauge. In this use, the transparent container 16 may be placed for example in a field. Here, an image 46 of rain 48 in the transparent container 16 can be captured by means of the camera 12 of the mobile computing device 10, wherein the app 30 determines the amount of rain 48 in the transparent container 16 based on that captured image 46. The amount of rain 48 in the transparent container 16 could be determined in the same way(s) as the layers 36a-c discussed above. The amount of rain 46 may for example be the volume of rain or the height H expressed in mm. The volume of rain may be calculated based on the height and the volume or the (base) area of the cylinder portion 20, which volume or (base) area may be known to the app 30.

Furthermore, push notifications could conveniently be sent to the mobile computing device 10 via the app 30 (and using the wireless communication means), prompting the user/famer to check the transparent container 16 based on a past weather forecast of rain, as illustrated by 50. Furthermore, the determined amount of rain could readily and automatically be fed back from the app 30 and mobile computing device 10 to a weather forecast provider by means of the wireless communication means, as illustrated by 52.

It may be an advantage to know the exact amount of rain that fell on a field to provide accurate fertilizer recommendations, for example in S7. A field well supplied in rain may require more fertilizer to obtain high yield. Over-abundant rain may also lead to nutrient leaching, i.e. excess water in the field may absorb nutrients present in the soil and carry these out of the field. Again, the app 30 may take this into consideration when delivering a fertilizer recommendation to the user.

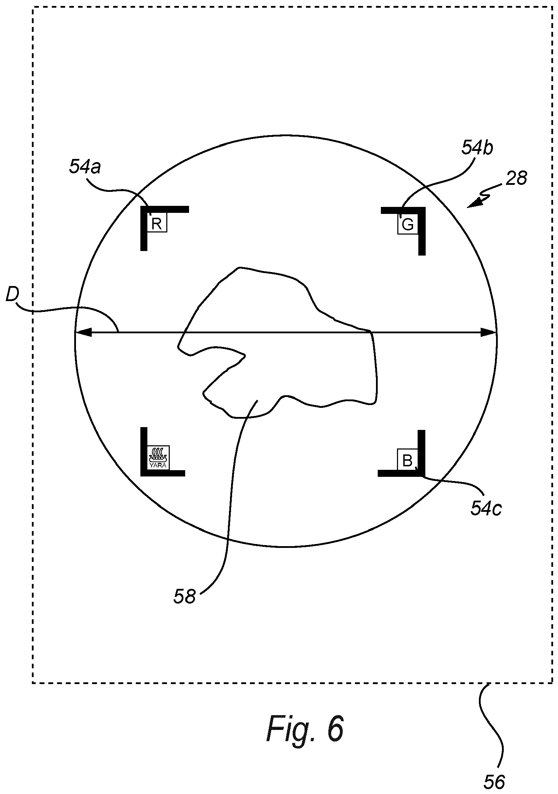

Moving on to fig. 6, the cap 28 for the transparent container 16 may have a colour chart known to the app 30. The colour chart may for example be a red marker 54a, a green marker 54b, and a blue marker 54c on the cap 28. Here, an image 56 of an object 58 with at least a portion of the cap 28 as background and with the markers 54a-c visible may be captured by means of the camera 12 of the mobile computing device 10, wherein the app 30 may assess the colour of the object 58 based on that captured image 56 taking into account those known colours of the cap 28. Assessing the colour of the object 58 may include detecting a colour difference between the colour chart as depicted in the captured image 56 and the known colour chart of the cap 28, and using the detected difference as calibration to assess the (true) colour of the object 58. If for example the imaged marker 54c comes off more bluish than specified, the app 30 may reduce the blue component of the imaged colour of the object 58 to determine or assess the true colour of the object 58. The object 58 could for example be soil (like soil 32), whereby the colour assessment can be combined with the determined soil texture and corresponding GPS coordinates (given by the GPS receiver of the mobile computing device 10). These data may in turn be used to build a soil colour-texture map.

Alternatively or complementary, the cap 28 for the transparent container 16 may have a size known to the app 30. The known size may for example be the diameter D of the cap 28. Here, an image (like image 56) of an object (like object 58) placed on the cap 28 and with at least a portion of the cap 28 as background and with the known size D visible may be captured by means of the camera 12 of the mobile computing device 10, wherein the app 30 may assess the size of the object 56 based on that captured image taking into account that known size D of the cap 28. For example, if the imaged object 58 has a width of 600 pixels and the imaged diameter D is 800 pixels, then the is assessed size (width) of the object 56 is (600/800)*10 cm=7.5 cm given a known size D=10 cm.

The person skilled in the art realizes that the present invention by no means is limited to the embodiments described above. On the contrary, many modifications and variations are possible within the scope of the appended claims.

Furthermore, both using the container as a rain gauge (e.g. claim 10) and using the cap for assessing colour and/or size of an object (e.g. claim 11, 12, 13) could be implemented independently of the soil texture determination.

## Claims

1. A method for determining soil texture, comprising:
placing soil (32) and a water-based liquid (34) in a substantially transparent container (16);
mixing the soil and water-based liquid in the substantially transparent container;
allowing particles of the soil to separate into layers (36a-c) by gravity;
capturing an image (38) of the layers in the substantially transparent container by means of a camera (12) of a mobile computing device (10); and
an app (30) of the mobile computing device detecting the layers in the captured image and determining the texture of the soil based on the detected layers.

2. A method according to claim 1, wherein detecting the layers in the captured image includes determining the thickness (42a-c) of each layer based on the captured image and determining the percentage of each layer based on the determined thicknesses.

3. A method according to claim 1 or 2, wherein detecting the layers in the captured image includes distinguishing at least one delimitation (44a-b) between the layers by identifying differences in colour between the layers.

4. A method according to any one of the preceding claims, wherein detecting the layers in the captured image includes distinguishing at least one delimitation (44a-b) between the layers by identifying differences in texture, in particular granularity, between the layers.

5. A method according to any one of the preceding claims, wherein said layers include a sand layer (36a), a silt layer (36b), and a clay layer (36c).

6. A method according to any one of the preceding claims, wherein the app displays on a display (14) of the mobile computing device a shape (40) corresponding to at least a portion of the shape of the substantially transparent container, wherein a user positions and/or aims the mobile computing device such that the substantially transparent container as seen in live view by the camera on the display fits the displayed shape, and wherein the image of the layers in the substantially transparent container is captured as the mobile computing device is so positioned and/or aimed.

7. A method according to any one of the preceding claims, wherein the substantially transparent container comprises a funnel portion (18) emptying into a cylinder portion (20) with a bottom (22).

8. A method according to any one of the preceding claims, further comprising:
the app providing a fertilizer recommendation based at least partly on the determined texture of the soil.

9. A method according to any one of the preceding claims, wherein the mobile computing device is a smartphone or a tablet.

10. A method according to any one of the preceding claims, further comprising:
using the substantially transparent container being emptied as a rain gauge;
capturing an image (46) of rain (48) in the substantially transparent container by means of the camera of the mobile computing device; and
the app determining the amount of rain in the substantially transparent container based on the captured image of rain in the substantially transparent container.

11. A method according to any one of the preceding claims, wherein the substantially transparent container is closed or closable by a cap (28) having at least one of a colour chart (54a-c) known to the app and a size (D) known to the app.

12. A method according to claim 11, further comprising:
capturing an image (56) of an object (58) with at least a portion of the cap as background by means of the camera of the mobile computing device; and
the app assessing the colour of the object based on the captured image of the object taking into account the known colour chart of the cap and/or assessing the size of the object based on the captured image of the object taking into account the known size of the cap.

13. A method according to claim 12, wherein assessing the colour of the object based on the captured image of the object taking into account the known colour chart of the cap includes detecting a colour difference between the colour chart of the cap as depicted in the captured image of the object and the known colour chart of the cap, and using the detected difference as calibration to assess the colour of the object.

14. A mobile computing device (10) for determining soil texture, which mobile computing device comprises a camera (12), wherein the mobile computing device is configured to:
instruct a user of the mobile computing device to capture an image (38) of soil layers (36a-c) in a substantially transparent container (16) using the camera;
detect the soil layers in a captured image (38) of the soil layers in the substantially transparent container; and
determine a soil texture based on the detected soil layers.

15. A computer program product (30) comprising computer program code to perform, when executed on a computer (10), the steps of:
receiving an image (38) of soil layers (36a-c) in a substantially transparent container captured by a camera (12) of a mobile computing device (10);
detecting the soil layers in the received image; and
determining a soil texture based on the detected soil layers.

16. A computer-readable storage medium comprising the computer program product according to claim 15.

17. An electrical signal embodied on a carrier wave and propagated on an electrical medium, the electrical signal comprising the computer program product according to claim 15.

18. A mobile computing device (10) comprising the computer-readable storage medium according to claim 16.

19. A mobile computing device according to claim 14 or 18 in combination with the substantially transparent container.
